# EUROPEAN PATENT APPLICATION

(11) **EP 4 356 903 A1**
(43) Date of publication of application: **24.04.2024**
(21) Application number: 23204447.9
(22) Date of filing: 18.10.2023
(51) Int. Cl.: A61K 9/20, A61K 9/28, A61K 9/48, A61K 31/4439

(54) **MICROTABLETS COMPRISING LANSOPRAZOLE**

(30) Priority: 19.10.2022 TR 202215877
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR); SARP, Onder, Istanbul (TR)
(74) Representative: Sevinç, Erkan

(57) **Abstract**

The present invention relates to pharmaceutical composition in the form of capsule having enteric coating microtablets comprising lansoprazole wherein microtablets further comprising at least one surfactant, at least one pH modifier and further at least other pharmaceutically excipient and wherein enteric coating comprising metacrylic acid copolymer dispersion. The capsule is obtained by a process which is free of solvent, the process is simple, rapid, cost effective, time-saving and industrially convenient process.

## Description

### Field of the invention

The present invention relates to pharmaceutical composition in the form of capsule having enteric coating microtablets comprising lansoprazole wherein microtablets further comprising at least one surfactant, at least one pH modifier and further at least other pharmaceutically excipient and wherein enteric coating comprising metacrylic acid copolymer dispersion. The capsule is obtained by a process which is free of solvent, the process is simple, rapid, cost effective, time-saving and industrially convenient process.

### Background of the invention

Lansoprazole, chemical name 2-(((3-methyl-4-(2,2,2-trifluoroethoxy)-2-pyridyl)methyl) sulfinyl)-1H-benzoyl Imidazole, brownish white crystalline powder, easily soluble in dimethylamide, soluble in methanol, insoluble in ethanol and ether, almost insoluble in water. It has a chemical structure which is shown in the Formula I.

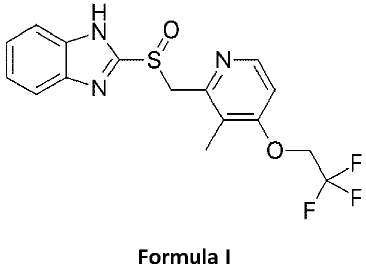

Lansoprazole is the second proton pump inhibitor anti-ulcer drug developed by Takeda Corporation of Japan after omeprazole.1992ln 1999, Japan's Takeda Pharmaceutical Co., Ltd. and Houde Company first officially put it on the market in France, and in May 1995, it was approved by the FDA to be listed in the United States.

Lansoprazole is extremely unstable under acidic conditions, and is degraded to inactive components in a short time under low pH environment, so it is easily degraded in gastric acid environment. In order to improve the bioavailability of the drug, it needs to be prepared as an enteric-coated preparation.

Chinese Patent Application No.: 201010245689.6 discloses a lansoprazole enteric capsule formulation and preparation method, using sodium bicarbonate as a stabilizer, cross-linked polyvinylpyrrolidone as a disintegrant, and acrylic resin as an enteric coating agent.

U.S. Patent No. 6,328,994 discloses an orally disintegrable lansoprazole tablet which comprises fine enteric coated granules having an average particle diameter of 400µπ 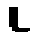 or less.

Therefore, it is still necessary to prepare the lansoprazole enteric-coated capsule that a kind of related substance content is low, the desired stability and is suitable for industrialized production.

### Detailed description of the Invention

The main object of the present invention is to eliminate problems caused by active substance and bringing additional advantages to the relevant prior art. To explain in more detail, the main purpose is to provide a pharmaceutical composition in the form of capsule having enteric coating microtablets having high physical and chemical stability and a long shelf life by the help of selection of excipients.

Another object of the present invention is to obtain a pharmaceutical composition in the form of capsule having enteric coating microtablets providing improved dissolution profile, it also is cost effective.

Another object of the present invention is to obtain a pharmaceutical composition in the form of capsule having enteric coating microtablets providing improved excellent pharmacomechanic properties, such as flowability, compressibility and content uniformity.

In particular, there is a need to combine an acid-labile physiologically active substance, with basic inorganic salts and so forth for stability, and further to coat with coating layers such as an enteric layer. It is an important problem to produce a small enteric coated microtablets, even though it contains the acid-labile physiologically active substance in small content. In this invention, a pharmaceutical composition in the form of capsule having enteric coating microtablets is used. The composition provides the desired stability and the desired profile. Also, microtablets have content uniformity and flowability.

The microtablets are cylindrical with a flat or convex upper side and lower side and with a diameter and height which are preferably approximately equal and, independently of one another, preferably have a tablet size of from about 1 mm to about 5 mm, more preferably from about 1.5 mm to about 4.5 mm. The microtablets have a tablet weight of about 1 mg to about 50 mg. Preferably, the microtablets have a tablet weight of about 2 mg to about 35 mg.

According to one embodiment of the present invention, a pharmaceutical composition in the form of capsule having enteric coating microtablets comprising lansoprazole wherein;
- Microtablets comprising at least one surfactant, at least one pH modifier and further at least other pharmaceutically excipient,
- Enteric coating comprising methacrylic acid copolymer dispersion.

An enteric coating protects the lansoprazole in microtablet from the acidic environment of the stomach, permitting it to be released in the higher pH environment of the lower GI tract. So, enteric coating provides to release the active ingredient gradually and predictably over a 12-hour to 24-hour period.

The methacrylic acid copolymer is used alone or in combination with other another enteric polymers to achieve the desired delayed release profile. It also helps to provide stability. Especially, Methacrylic Acid-Ethyl Acrylate Copolymer (1:1) Type A is used.

According to one embodiment of the present invention, using Ph modifier ensures that lansoprazole, which is unstable at low pH, remains at the desired stability under long conditions.

According to one embodiment of the present invention, the amount of lansoprazole is between 3.0 % and 20.0% by weight in the total composition. Preferably, the amount of lansoprazole is between 5.0 % and 13.0% by weight in the total composition.

Suitable surfactants are selected from the group comprising sodium dodecyl sulphate, sodium lauryl sulfate, cetylpyridinium chloride, docusate sodium, lauric acid, polyoxyethylene sorbitan fatty acid esters (polysorbate), phospholipids, cetrimide or mixtures thereof.

According to one embodiment of the present invention, the surfactant is sodium dodecyl sulphate. It is a synthetic organic compound widely used in pharmaceutical products as an anionic surfactant. In the present invention, it provides the desired profile. It also helps to provide flowability.

According to one embodiment of the present invention, the amount of surfactant is between 0.1 % and 3.0% by weight in the total composition.

Suitable pH modifiers are selected from the group comprising magnesium carbonate, aluminum potassium sulfate, anhydrous citric acid, anhydrous disodium hydrogen phosphate, potassium carbonate, anhydrous sodium dihydrogen phosphate, dibasic potassium sulfate, calcium carbonate, dilute hydrochloric acid, monobasic potassium phosphate, phosphoric acid, adipic acid, sodium acetate, sodium bicarbonate, sodium carbonate, sodium citrate, sodium dihydrogen phosphate dihydrate, tribasic sodium phosphate or mixtures thereof.

According to one embodiment of the present invention, the pH modifier is magnesium carbonate.

According to one embodiment of the present invention, the amount of pH modifier is between 1.0 % and 8.0% by weight in the total composition.

According to one embodiment of the present invention, the microtablets comprise further at least one pharmaceutically acceptable excipient which is selected from the group comprising diluents, disintegrants, binders, lubricants, glidants or mixtures thereof.

Suitable diluents are selected from the group comprising microcrystalline cellulose, mannitol, lactose monohydrate, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment of the present invention, the diluent is microcrystalline cellulose or mannitol or lactose monohydrate or mixtures thereof.

According to one embodiment of the present invention, the diluent is microcrystalline cellulose.

According to one embodiment of the present invention, the diluent is mannitol and lactose monohydrate.

According to one embodiment of the present invention, the amount of diluents is between 30.0 % and 75.0% by weight in the total composition. Preferably, the amount of diluents is between 40.0 % and 60.0% by weight in the total composition. The amount provides the desired content uniformity. Since the amount of active ingredient is low compared to the whole composition, it is important to ensure content uniformity.

Suitable disintegrants are selected from the group comprising croscarmellose sodium, crospovidone, alginic acid, alginates, ion-exchange resins, magnesium aluminium silica, sodium carboxymethyl cellulose, calcium silicate, sodium starch glycolate, carboxymethyl cellulose, calcium carboxymethyl cellulose, docusate sodium, guar gum, low substituted hydroxypropyl cellulose, polyacryline potassium, poloxamer, povidone, sodium alginate, sodium glycine carbonate, or mixtures thereof.

According to one embodiment of the present invention, the disintegrant is croscarmellose sodium or crospovidone or mixtures thereof.

According to one embodiment of the present invention, the amount of disintegrant is between 1.0 % and 8.0% by weight in the total composition.

Suitable binders are selected from the group comprising hydroxypropyl cellulose, polyvinylpyrrolidone, natural gums, gelatin, collagen, agar, alginates, co-povidone, sodium alginate, gum, guar gum, starch mucilage, acacia mucilage, bentonite, laponit, cetostearyl alcohol, pullulan, polydextrose, polyethylene oxide, pectin, aluminia hydroxide, hyaluronic acid or mixtures thereof.

According to one embodiment of the present invention, the binder is hydroxypropyl cellulose or polyvinylpyrrolidone or mixtures thereof.

According to one embodiment of the present invention, the amount of binder is between 0.1 % and 5.0% by weight in the total composition.

Suitable glidants are selected from the group comprising talc, colloidal silica anhydrous, colloidal silicon dioxide, calcium silicate, aluminium silicate, magnesium silicate, magnesium oxide, starch or mixtures thereof.

According to one embodiment of the present invention, the glidant is talc or colloidal silica anhydrous or mixtures thereof. Using these glidants provides the flowability.

According to one embodiment of the present invention, the amount of glidant is between 0.1 % and 9.0% by weight in the total composition.

Suitable lubricants are selected from the group comprising magnesium stearate, silica, calcium stearate, stearic acid, polyethylene glycol, sodium stearyl fumarate, sodium lauryl sulfate, magnesium lauryl sulfate, fumaric acid, glyceryl palmito sulfate, hydrogenated vegetable oil, zinc stearate or mixtures thereof.

According to one embodiment of the present invention, the lubricant is magnesium stearate.

According to one embodiment of the present invention, the amount of lubricant is between 0.4 % and 3.0% by weight in the total composition.

According to one embodiment of the present invention, microtablets comprises;
- Lansoprazole
- Mannitol
- Magnesium carbonate
- Lactose monohydrate
- HPC
- Sodium dodecyl sulphate

According to one embodiment of the present invention, microtablets comprises;
- Lansoprazole
- Microcrystalline cellulose pH 112
- Magnesium carbonate
- PVP K-30
- Sodium dodecyl sulphate
- Crospovidon CL

According to one embodiment of the present invention, the enteric coating comprises;
- Metacrylic acid copolymer dispersion,
- Sodium hydroxide,
- Polysorbate 80.

However, lansoprazole is unstable and prone to impurities. In addition to affecting the efficacy of drugs, impurities may increase the risk of toxic and side effects. In this invention, to eliminate this problem, a process which is free of solvent is preferred in terms of the desired stability. In using process, lansoprazole is not exposed to moisture and solvents. Thanks to this way eliminates the problems stability of lansoprazole and provides additional advantages to the relevant field of art.

According to one embodiment of the present invention, the microtablets are obtained by a process which is free of solvent.

The present invention is to obtain stable microtablets which is obtained by dry granulation or direct compression with improved stability.

### Example 1: Microtablets are obtained by direct compression

| **Ingredients** | | **Amount (% by weight of the total formulation)** | |
|---|---|---|---|
| Lansoprazole | | 3.0 - 15.0 | |
| Mannitol | | 40.0 - 57.0 | |
| Magnesium carbonate | | 1.0 - 8.0 | |
| Lactose monohydrate | | 1.0 - 8.0 | |
| HPC | | 0.1 - 5.0 | |
| Sodium dodecyl sulphate | | 0.1 - 3.0 | |
| Croscarmellose sodium | | 1.0 - 8.0 | |
| Talc | | 0.1 - 4.5 | |
| Colloidal silica anhydrous | | 0.1 - 4.5 | |
| Magnesium stearate | | 0.4 - 3.0 | |
| | **Microtablet** | | |
| Enteric coating | | 10.0 -30.0 | |
| **Total Enteric coating microtablet** | | | **100** |

### Example 2: Microtablets are obtained by direct compression

| **Ingredients** | | **Amount (% by weight of the total formulation)** | |
|---|---|---|---|
| Lansoprazole | | 8.5 | |
| Mannitol | | 49.2 | |
| Magnesium carbonate | | 3.1 | |
| Lactose monohydrate | | 3.7 | |
| HPC | | 0.7 | |
| Sodium dodecyl sulphate | | 0.7 | |
| Croscarmellose sodium | | 3.0 | |
| Talc | | | 3.90 |
| Colloidal silica anhydrous | | 3.90 | |
| Magnesium stearate | | 1.30 | |
| | **Microtablet** | | |
| Enteric coating | | 22 | |
| **Total Enteric coating microtablet** | | | **100** |

A process for example 1 or 2;
a) Mixing Lansoprazole, Mannitol, Magnesium carbonate, Lactose monoydrate, HPC, Sodium dodecyl sulphate, croscarmellose sodium, colloidal silica, talc for 15 minutes,
b) Adding weighed and 0.6 mm sieved Magnesium stearate to dry mixture and mix for 3 minutes,
c) Obtaining microtablets,
d) Coating the microtablets with enteric coating having Metacrylic acid copolymer dispersion,
e) Filling the microtablets into capsules.

### Example 3: Microtablets are obtained by dry granulation

| **Ingredients** | | **Amount (% by weight of the total formulation)** | |
|---|---|---|---|
| Lansoprazole | | 3.0-15.0 | |
| Microcrystalline cellulose pH 112 | | 40.0 - 65.0 | |
| Magnesium carbonate | | 1.0 -8.0 | |
| PVP K-30 | | 0.1 - 5.0 | |
| Sodium dodecyl sulphate | | 0.1 - 3.0 | |
| Crospovidon CL | | 1.0 - 8.0 | |
| Colloidal silica anhydrous | | 0.1 - 3.0 | |
| Magnesium stearate | | 0.4 - 3.0 | |
| | **Microtablet** | | |
| Enteric coating | | 10.0 -30.0 | |
| **Total Enteric coating microtablet** | | | **100** |

### Example 4: Microtablets are obtained by dry granulation

| **Ingredients** | | **Amount (% by weight of the total formulation)** | |
|---|---|---|---|
| Lansoprazole | | 8.5 | |
| Microcrystalline cellulose pH 112 | | 56.13 | |
| Magnesium carbonate | | 3.97 | |
| PVP K-30 | | 3.40 | |
| Sodium dodecyl sulphate | | 0.99 | |
| Crospovidon CL | | 3.97 | |
| Colloidal silica anhydrous | | 0.57 | |
| Magnesium stearate | | 0.57 | |
| | **Microtablet** | | |
| Enteric coating | | 21.9 | |
| **Total Enteric coating microtablet** | | | **100** |

A process for example 3 or 4;
a) Mixing Lansoprazole, a half of MCC pH 112, colloidal silicon dioxide, PVP K-30, magnesium carbonate, sodium dodecyl sulphate for 10 minutes,
b) Adding weighed and 0.6 mm sieved a half of Magnesium stearate to dry mixture and mix for 3 minutes,
c) Performing slug granulation in compactor,
d) Sieving dry granules into 1.2 mm,
e) Adding the other half of MCC pH 112, Crospovidon CL and then mixing for 5 minutes,
f) Adding 0.6mm sieved the other half of Mg-stearate and mixing for 3 minutes,
g) Obtaining microtablets,
h) Coating the microtablets with enteric coating having Metacrylic acid copolymer dispersion,
i) Filling the microtablets into capsules.

### Enteric coating

| **Ingredients** |
|---|
| Metacrylic acid copolymer dispersion |
| Sodium hydroxide |
| Polysorbate 80 |
| Propilen glikol |
| Titanium dioxide |
| Cetyl alcohol |

## Claims

1. A pharmaceutical composition in the form of capsule having enteric coating microtablets comprising lansoprazole wherein;
- Microtablets comprising at least one surfactant, at least one pH modifier and further at least other pharmaceutically excipient,
- Enteric coating comprising methacrylic acid copolymer dispersion.

2. The pharmaceutical composition according to claim 1, wherein surfactants are selected from the group comprising sodium dodecyl sulphate, sodium lauryl sulfate, cetylpyridinium chloride, docusate sodium, lauric acid, polyoxyethylene sorbitan fatty acid esters (polysorbate), phospholipids, cetrimide or mixtures thereof.

3. The pharmaceutical composition according to claim 1, wherein the surfactant is sodium dodecyl sulphate.

4. The pharmaceutical composition according to claim 1, wherein pH modifiers are selected from the group comprising magnesium carbonate, aluminum potassium sulfate, anhydrous citric acid, anhydrous disodium hydrogen phosphate, potassium carbonate, anhydrous sodium dihydrogen phosphate, dibasic potassium sulfate, calcium carbonate, dilute hydrochloric acid, monobasic potassium phosphate, phosphoric acid, adipic acid, sodium acetate, sodium bicarbonate, sodium carbonate, sodium citrate, sodium dihydrogen phosphate dihydrate, tribasic sodium phosphate or mixtures thereof.

5. The pharmaceutical composition according to claim 1, wherein the pH modifier is magnesium carbonate.

6. The pharmaceutical composition according to claim 1, wherein the microtablets comprise further at least one pharmaceutically acceptable excipient which is selected from the group comprising diluents, disintegrants, binders, lubricants, glidants or mixtures thereof.

7. The pharmaceutical composition according to claim 6, wherein diluents are selected from the group comprising microcrystalline cellulose, mannitol, lactose monohydrate, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

8. The pharmaceutical composition according to claim 7, wherein the diluent is microcrystalline cellulose or mannitol or lactose monohydrate or mixtures thereof.

9. The pharmaceutical composition according to claim 8, wherein the amount of diluents is between 30.0 % and 75.0% by weight in the total composition.

10. The pharmaceutical composition according to claim 6, wherein disintegrants are selected from the group comprising croscarmellose sodium, crospovidone, alginic acid, alginates, ion-exchange resins, magnesium aluminium silica, sodium carboxymethyl cellulose, calcium silicate, sodium starch glycolate, carboxymethyl cellulose, calcium carboxymethyl cellulose, docusate sodium, guar gum, low substituted hydroxypropyl cellulose, polyacryline potassium, poloxamer, povidone, sodium alginate, sodium glycine carbonate, or mixtures thereof.

11. The pharmaceutical composition according to claim 6, wherein binders are selected from the group comprising hydroxypropyl cellulose, polyvinylpyrrolidone, natural gums, gelatin, collagen, agar, alginates, co-povidone, sodium alginate, gum, guar gum, starch mucilage, acacia mucilage, bentonite, laponit, cetostearyl alcohol, pullulan, polydextrose, polyethylene oxide, pectin, aluminia hydroxide, hyaluronic acid or mixtures thereof.

12. The pharmaceutical composition according to claim 11, wherein the binder is hydroxypropyl cellulose or polyvinylpyrrolidone or mixtures thereof.

13. The pharmaceutical composition according to claim 6, wherein glidants are selected from the group comprising talc, colloidal silica anhydrous, colloidal silicon dioxide, calcium silicate, aluminium silicate, magnesium silicate, magnesium oxide, starch or mixtures thereof.

14. The pharmaceutical composition according to claim 1, wherein the enteric coating comprises;
- Metacrylic acid copolymer dispersion,
- Sodium hydroxide,
- Polysorbate 80.

15. The pharmaceutical composition according to claim 1, wherein the microtablets are obtained by a process which is free of solvent.
